# EUROPÄISCHE PATENTSCHRIFT

(11) **EP 2 066 622 B1**
(45) Veröffentlichungstag und Bekanntmachung des Hinweises auf die Patenterteilung: **06.11.2013**
(21) Anmeldenummer: 06793479.4
(22) Anmeldetag: 13.09.2006
(51) Int. Cl.: C07C 277/08, C07C 279/28

(54) **VERFAHREN ZUR HERSTELLUNG VON HEXAMETHYLENBISCYANOGUANIDIN UND CHLORHEXIDIN**
PROCESS FOR PREPARING HEXAMETHYLENEBISCYANOGUANIDINE AND CHLORHEXIDINE
PROCÉDÉ DE FABRICATION D'HEXAMÉTHYLÈNEBISCYANOGUANIDINE ET DE CHLORHEXIDINE

(43) Veröffentlichungstag der Anmeldung: 10.06.2009
(73) Patentinhaber: Evonik Degussa GmbH, 45128 Essen (DE)
(72) Erfinder: WERLE, Peter, 63571 Gelnhausen (DE); MERZ, Friedhelm, 55283 Nierstein (DE); TRAGESER, Martin, 63571 Gelnhausen-Höchst (DE)
(86) Internationale Anmeldenummer: PCT/EP2006/066318
(87) Internationale Veröffentlichungsnummer: WO 2008/031456

(56) Entgegenhaltungen:
- EP-A1- 0 305 721

## Beschreibung

Die Erfindung betrifft ein verbessertes Verfahren zur Herstellung von Hexamethylenbiscyanoguanidin der Formel (I)und Chlorhexidin-Base der Formel (II).

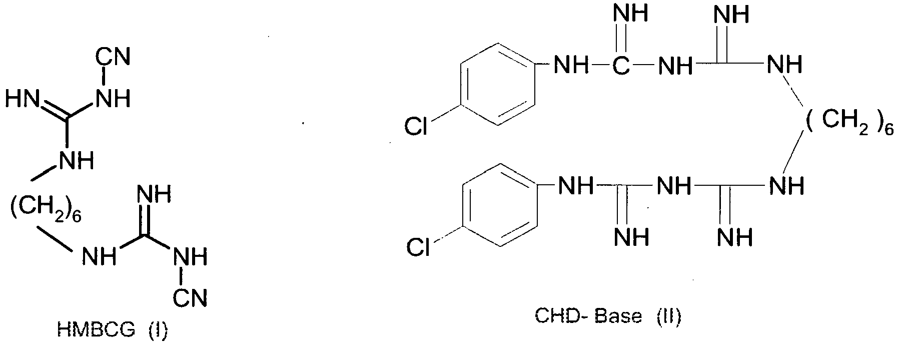

Hexamethylenbiscyanoguanidin (HMBCG) ist eine Vorstufe, welche für die Herstellung von Chlorhexidin verwendet wird. Chlorhexidin wiederum ist eine basisch wirkende Substanz, die überwiegend in Form ihrer Salze und hier vor allem in Form des wasserlöslichen Salzes der Gluconsäure verwendet wird. Chlorhexidin-Salze stellen bedeutende antibakterielle Substanzen dar, sowohl für den Einsatz im Human- als auch im Tierbereich. Hervorzuheben sind die niedrige Toxizität und allgemeine Verträglichkeit mit kationischen und anionischen Detergentien. Das Chlorhexidindigluconat wird als 20 %ige wäßrige Lösung angeboten und ist die derzeit einzige kommerziell verfügbare wasserlösliche Form der Base. Flüssige Formulierungen des Chlorhexidindigluconats (CHD-Gluconat) werden vielfältig modifiziert und als antibakterieller Zusatz in Kosmetika, zur Hautdesinfektion, Wundbehandlung in der Veterinärmedizin als Euterdesinfektibnsmittel und auch zur Oberflächendesinfektion eingesetzt.

In der EP305721 wird die Synthese von Chlorhexidin beschrieben. Diese erfolgt zweistufig (Schema 1). In der ersten Stufe des Verfahrens werden Natriumdicyanamid mit Hexmethylendiamindihydrochlorid in alkoholischer Lösung mit Natriumdicyanamid zu Hexamethylenbiscyanoguanidin umgesetzt. Da die Umsetzung zum HMBCG bei Temperaturen >110° bevorzugt abläuft, wird entsprechend hochsiedendes Butanol als Lösungsmittel eingesetzt. Anschließend wird das NDCA zugegeben und die Mischung 2 - 4 h am Rückfluß erhitzt. Zugabe von Wasser und gleichzeitige Destillation von Butanol führt zu einem Austausch des Lösungsmittels im Reaktor, das HMBCG fällt vollständig aus, das gebildete Natriumchlorid (s. Schema 1) wird gelöst und bei der nachfolgenden Filtration mit der Mutterlauge entsorgt. Die 1. Stufe läuft nur dann mit zufriedenstellenden Ausbeuten, wenn ein Katalysator zugesetzt wird. Die EP 305721 schlägt vorzugsweise Triethylamin in diesem Zusammenhang vor, um einen pH von etwa 9 einzustellen.

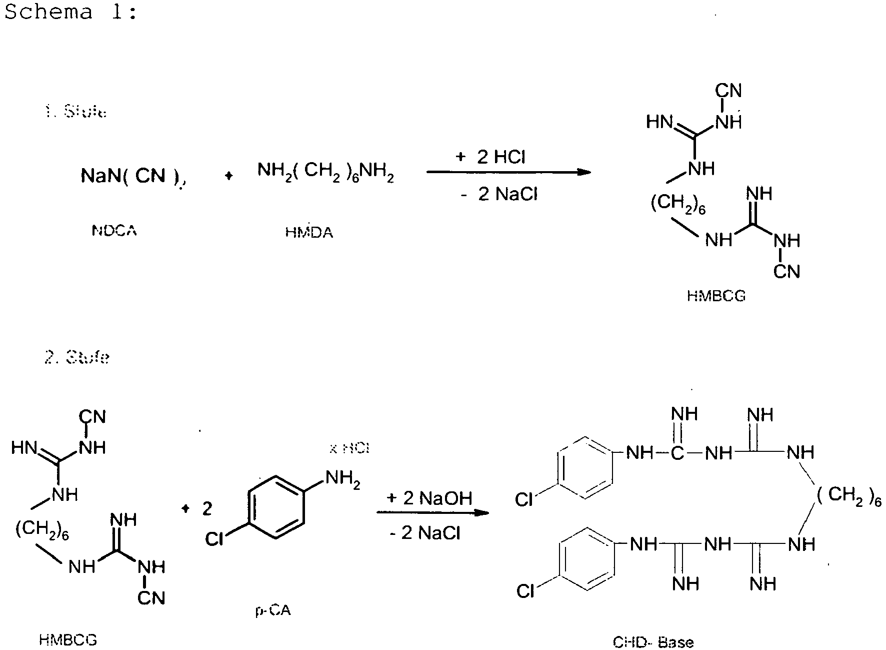

Das so hergestellte HMBCG kann in einem 2. Verfahrensschritt (s.o.) durch Umsetzen mit 4-Chloranilinhydrochlorid in einem alkoholischen Lösungsmittel wie Ethanol, n- bzw. iso-Propanol, oder 2 Ethoxyethanol am Rückfluss zum Chlorhexidinhydrochlorid umgesetzt werden, das nach Abkühlen, Filtrieren und Trocknen zu isolieren ist. Durch Zugabe von NaOH-Lauge kann daraus aber auch die Chlorhexidin-Base freigesetzt werden, wie dies in der brasilianischen Patentanmeldung BR9300129 beschrieben ist. Weiterhin ist auch die Zugabe von heißer, wässriger NaOH zur heißen Reaktionslösung möglich, wobei die Base sofort freigesetzt wird und in der alkoholischen Lösung verbleibt, während das gebildete Kochsalz sich in dem sich ausbildenden zweiphasigen System in der unteren wässrigen Phase löst. Beide Phasen können leicht separiert werden; aus der alkoholischen kristallisiert während des Abkühlens die freie Base aus, die anschließend ggf. weiteren Reinigungsprozessen unterworfen werden kann.

Nachteilig bei dem Verfahren sind die großen Mengen an Wasser und verschiedenen Lösungsmittel, die destilliert werden müssen und die zu niedrigen Raum-Zeit Ausbeuten führen, bzw. schwierige Recyclierungsprozedere bedingen.

Aufgabe der vorliegenden Erfindung war es deshalb ein Verfahren zur Herstellung von Hexamethylenbiscyanoguanidin anzugeben, welches im technischen Maßstab bevorzugt eingesetzt werden kann. Insbesondere sollte das vorliegende Verfahren unter ökologischen wie ökonomischen Gesichtspunkten den Verfahren des Standes der Technik überlegen sein. Speziell der Einsatz von unterschiedlichen organischen Lösungsmitteln sollte möglichst vermieden werden. Weiterhin war es ein Anliegen der Erfindung, dass der gesamte Prozess zur Herstellung von Chlorhexidin-Base ohne Zugabe weiterer nicht produktrelevanter chemischer Substanzen auskommen, welche im nachhinein wieder mühsam vom Produkt getrennt werden müssen.

Diese und weitere nicht genannte, sich jedoch aus dem Stand der Technik in naheliegender Weise ergebenden Aufgaben werden durch ein Verfahren mit den Merkmalen des gegenständlichen Anspruch 1 gelöst. Abhängige Ansprüche 2 bis 5 bilden bevorzugte Ausführungsformen des erfindungsgemäßen Verfahrens. Ansprüche 6 und 7 richten sich auf ein Verfahren zur Herstellung von Chlorhexidin-Base.

Dadurch, dass man in einem Verfahren zur Herstellung von Hexamethylenbiscyanoguanidin der Formel (I) 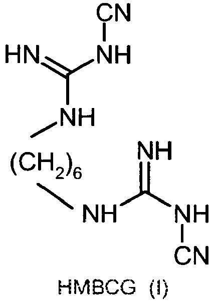 die Umsetzung von Hexamethylendiamin oder seines Salzes mit Natriumdicyanamid in einem alkoholischen Lösungsmittel in Gegenwart katalytischer Mengen an Chlorhexidin-Base durchführt, gelangt man in äußerst einfacher, dafür aber nicht minder überraschender bzw. naheliegender Weise zur Lösung der gestellten Aufgaben. Durch Anwendung des erfindungsgemäßen Verfahrens gelingt es, die vorstehend beschriebenen Verfahrensstufen (Schema 1) zu einem Verfahrensschritt zusammenzufassen und unter Verwendung nur eines für beide Stufen gleichermaßen geeigneten Lösungsmittels auszukommen. Darüber hinaus wird die aufwendige Destillation von Wasser bzw. Wasser-Butanolmischungen vermieden und die Isolierung der Zwischenstufe HMBCG kann unterbleiben.

In einer Ausführungsform kann das zur Reaktion eingesetzte Hexamethylendiamin als freie Base eingesetzt werden. Bevorzugt ist jedoch die Ausführungsform, in der das Hexamethylendiamin in Form seines Salzes mit Anionen starker Säuren zur Reaktion eingesetzt wird. Eine Variante geht dabei von der freien Base aus, welche vor der eigentlichen Umsetzung mit Natriumdicyanamid durch Umsetzung mit einer wasserfreien Säure mit einem pks-Wert <1, vorzugsweise <0 in das entsprechende Salz überführt wird. Als bevorzugte Säuren kommen hier insbesondere HCl, HBr (flüssig in Bomben erhältlich), Trichloressigsäure, Trifluoressigsäure, Methansulfonsäure, Methandisulfonsäure, Schwefelsäure in Betracht. Da Salze des Hexamethylendiamins kommerziell nicht leicht verfügbar sind und überdies wesentlich teurer als die Komponenten Hexamethylendiamin und Salzsäure sind, wird das Hydrochlorid in ganz besonders bevorzugter Weise in situ hergestellt.

In dem erfindungsgemäßen Verfahren wird Chlorhexidin-Base, welches das Produkt des gesamt Verfahrens darstellt, als Katalysator für die Umsetzung von Hexamethylendiamin und Natriumdicyanamid eingesetzt. Dieser Katalysator wird vorzugsweise in einer Menge von 0,0001 Mol% - 0,1 Mol%, bevorzugt 0,001 Mol% - 0,05 Mol% und besonders bevorzugt von 0,01 ± 0,005 Mol% bezogen auf eingesetztes Hexamethylendiamin eingesetzt. Der Vorteil des Einsatzes von Chlorhexidin-Base als Katalysator liegt darin, dass die nur begrenzte Löslichkeit der Base in den verwendbaren Alkoholen in Kombination der substanzeigenen Basizität es erlauben, offensichtlich einen derart günstigen pH-Wert im System einzustellen, dass die Reaktion in überaus vorteilhafter Art und Weise durchgeführt werden kann. Dabei kann überschüssige Base ungelöst im System verbleiben und bei der Aufarbeitung zurückgewonnen werden. Versehentlich überschüssig zugesetzte Säure wird neutralisiert und als Chlorhexidinsalz ausgetragen. Auf diese Art und Weise ist es möglich, ohne Zusatz weiterer Katalysatoren (wie z. B. Triethylamin), welche nach der Umsetzung mühsam vom Produkt zu trennen wären, im erfindungsgemäßen Verfahren auszukommen.

Weiterhin ist es vorteilhaft, die erfindungsgemäße Umsetzung des Hexamethylendiamins mit dem Natriumdicyanamid in einem Temperaturintervall von 100°C bis 140°C, vorzugsweise 105°C bis 130°C und ganz besonders bevorzugt von 120 ± 10°C zu betreiben.

Bevorzugt setzt man bei der erfindungsgemäßen Reaktion als alkoholisches Lösungsmittel Alkohole ausgewählt aus der Gruppe bestehend aus Methanol, Ethanol, Propanol und Isopropanol oder deren Gemische ein. Bei Einsatz kurzkettiger aliphatischer Alkohole liegen diese bei den angegebenen bevorzugten Temperaturen der Reaktion gasförmig vor. Es ist deshalb notwendig die Reaktion in einem Druckreaktor (z. B. Autoklaven) durchzuführen. Vorzugsweise kann die betrachtete Umsetzung beim sich einstellenden Eigendruck durchgeführt werden. Unter alkoholischen Lösungsmittel wird insbesondere ein solches verstanden, welches den oder die entsprechenden Alkohole als Hauptbestandteil der (> 50 Gew.-%) enthält. Weitere Bestandteile können Wasser und/oder andere organische Lösungsmittel sein.

Eine weitere Ausgestaltung der vorliegenden Erfindung beschäftigt sich mit einem Verfahren zur Herstellung von Chlorhexidin-Base, bei dem man das HMBCG wie oben angegeben erfindungsgemäß darstellt und dieses anschließend mit p-Chloranilin umsetzt.
Dabei ist der Einsatz der Base Chlorhexidin als Katalysator in der Synthese des HMBCGs besonders angezeigt, da dann das Chlorhexidin ohne Zwischenisolierung des HMBCGs hergestellt werden kann. Durch den möglichen Einsatz des Chlorhexidins als basischem Katalysator ist der Fachmann daher in der Lage, ein Eintopfverfahren zur Herstellung von Chlorhexidin zu etablieren, was unter ökonomischen wie ökologischen Gesichtspunkten besonders vorteilhaft ist, werden doch ansonsten notwendige Isolierungs- und Reinigungsschritte und der Einsatz weiterer organischer Lösungsmittel eingespart.

Beim hier beschriebenen erfindungsgemäßen Verfahren kann der Fachmann in geeigneter Art und Weise wie folgt vorgehen:
Hexamethylendiamin wird in einem alkoholischen Lösungsmittel vorgelegt. Das Lösungsmittel sollte für die Verfahrensschritte 1 und 2 (s. Schema 1) gleich gut geeignet und zur besseren Abtrennung vom Zielprodukt wasserlöslich sein. Es kommen hier vornehmlich niedrig siedende Alkohole wie Methanol, Ethanol sowie n- und iso-Propanol in Frage. Besonders bevorzugt wird iso-Propanol verwendet. Da für die Synthese des HMBCG Temperaturen von > 110°C bevorzugt sind, wird in dieser Stufe unter Druck (2-5 bar) gearbeitet.

Die Reaktion von Natriumdicyanamid (NDCA) mit einem Salz des HMDA verläuft wie beschrieben bevorzugt dann unter Bildung von Hexamethylenbiscyanoguanidin (HMBCG) ab, wenn das Salz des Hexamethylendiamins mit einer ausreichend starken Saure mit einem pks-Wert kleiner 1, vorzugsweise kleiner 0, eingesetzt wird.
Im allgemeinen wird die entsprechende Menge Säure zu einer vorgelegten alkoholischen Lösung des Hexamethylendiamins zugegeben und die Reaktionswärme abgeführt. Im Falle der Verwendung von Chlorwasserstoff fällt das entsprechende Dihydrochlorid in feiner, sehr reaktiver Verteilung an.

Die Umsetzung von Hexamethylendiamin (HMDA) x 2HCl mit Natriumdicyanamid (NDCA) findet vorzugsweise in einem engen pH-Intervall mit hervorragender Ausbeute statt. Bei pH-Werten < 7 werden zunehmend polymere Produkte (Polyhexamethylenbiguanidine) gebildet, bei höheren pH-Werten > 10 werden ebenfalls geringere Mengen an HMBCG gebildet. Die an sich einfach erscheinende Aufgabe, den pH-Wert des Systems durch Zugabe alkalischer "Katalysatoren" auf Werte um 9 einzustellen, erweist sich technisch als ausgesprochen problematisch. Grund dafür ist einmal die Schwierigkeit einer pH-Wert-Bestimmung in org. Medien und weiterhin das Fehlen einer Separationsstufe (Isolierung und Waschung von HMBCG), wobei die Katalysatoren entfernt werden können. Bei dem erfindungsgemäßen Verfahren verbleiben sie nämlich im Endprodukt und müssten dort durch geeignete zusätzliche Maßnahmen entfernt werden. Anorganische Stoffe wie NaOH erweisen sich wegen der hohen Basizität als problematisch in der Dosierung und führen leicht zu ungewolltem Abdriften des pH-Wertes, was einer sicheren Betriebsführung nicht förderlich ist. Chlorhexidin als Base bietet hier einen überraschend einfachen Ausweg. Es lässt sich nicht vollständig im angegebenen Lösungsmittelsystem lösen und bietet trotzdem milde basischen Bedingungen an, welche die Umsetzung von Natriumdicyanamid und Hexamethylendiamin fördern Eine Abtrennung des Katalysators ist darüber hinaus nicht notwendig, da er das eigentliche Verfahrens Endprodukt darstellt. Somit kann er vollständig im System verbleiben.

Nach dem Reaktionsende der ersten Stufe (HMBCG Bildung), die wie gesagt bei etwa 115 - 120°C verläuft, wird vorzugsweise die Temperatur reduziert (auf ca. 90 - 110°C) und p-Chloranilin (p-CA) sowie wässrige Salzsäure in der zur Umsetzung zum Chlorhexidinhydrochlorid benötigten Menge zugesetzt. Das kann durch Einpumpen erfolgen. Die Wassermenge ist nicht kritisch, da die CHD x 2HCl Bildung auch in wässrigen Systemen abläuft. Bevorzugt ist jedoch eine Menge an Wasser im Reaktionsmedium von 5 - 40 Gew.-%, vorzugsweise 15 - 30 Gew.-% und ganz besonders bevorzugt 20 - 25 Gew.-%. Alternativ kann auch festes p-CA x HCl zugegeben werden.

Die vorliegende Erfindung erlaubt in sehr eleganter dafür aber nicht minder naheliegender Weise, die Synthese des Hexamethylendiaminbiscyanoguanidins (HMBCG) in einem technischen Prozess unter ökonomischen und ökologischen Gesichtspunkten vorteilhaft durchzuführen. So konnte durch die geeignete Kombination von eingesetzter Base und Lösungsmittel erreicht werden, dass die vormals in zwei Stufen ablaufende Reaktion zum Chlorhexidin (Schema 1) nunmehr in einer Stufe ohne Zwischenisolierung des Hexamethylendiaminbiscyanoguanidins durchgeführt werden kann. Schwierige Produktaufreinigungen und Rezyklierung von organischen Lösungsmittel bzw. deren Entwässerung sind wesentlich vereinfacht worden. Überraschenderweise leidet darunter die Produktausbeute und Produktqualität in keinster Weise. Man erhält mit dem angegebenen Verfahren Ausbeuten von an die 70% über beide Stufen gerechnet. Ein Verfahren wie vorliegend ist durch den Stand der Technik in keinster Weise nahegelegt.

### Beispiel :

In einem geeigneten 3 - 5 l Druckreaktor werden 129, 1 g Hexamethylendiamin (90%ig; MG 116,2) zu vorgelegten 1450 ml Isopropanol gegeben, und unter Solekühlung werden bei 20 - max. 60°C 73,0 g HCl-Gas (MG.36,46) unter die Oberfläche dosiert. Nach beendeter Reaktion wird die Reaktionsmischung auf pH 7 eingestellt. Zu der feinen kristallinen Suspension werden nun 5,0 g (0,01 Mol) Chlorhexidin-Base und 184,0 g Natriumdicyanamid (97 %; MG 89,03) gegeben. Die Mischung wird 2 h unter Eigendruck bei 120 °C ( 3 - 4 bar) gerührt.

Nach dem Abkühlen auf ca 90°C werden zu der gebildeten HMBCG Suspension, eine Lösung bestehend aus 245 g p-Chloranilin (MG 127,57) in 360 ml Wasser und 175 ml Salzsäure (37%ig) gegeben, und anschließend wird die Mischung erhitzt. Nach 2 h bei 120°C unter Eigendruck (2,4 - 2,8 bar) wird die Lösung auf ca 80°C abgekühlt und durch Zugabe von 140 ml Natronlauge (40%ig) und 720 ml heißem Wasser die freie Base gebildet und die Phasentrennung eingeleitet.

Die untere wässrige Phase enthält das aus dem Prozess gebildete Salz und wird entsorgt, aus der org. Phase wird durch Abkühlen die Chlorhexidin-Base auskristallisiert und isoliert.
- Ausbeute :: 351,3 g Chlorhexidin-Base roh, trocken
Das entspricht 69,5 % d. Th.

Die Rohbase kann nun einer üblichen Reinigungsstufe (Umkristallisation) zugeführt, und/oder es kann die Umarbeitung ins Endprodukt (z.B. Gluconsäureaddukt) erfolgen.

## Patentansprüche

1. Verfahren zur Herstellung von Hexamethylenbiscyanoguanidin der Formel (I) 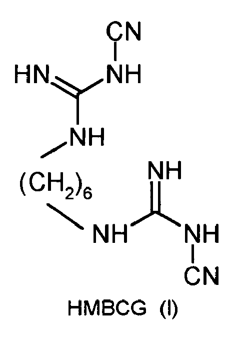 durch Umsetzung von Hexamethylendiamin oder seines Salzes mit Natriumdicyanamid in einem alkoholischen Lösungsmittel in Gegenwart katalytischer Mengen an Chlorhexidin-Base in einem Verfahrensschritt.

2. Verfahren nach Anspruch 1,
**dadurch gekennzeichnet, dass**
man das Salz des Hexamethylendiamins durch dessen Umsetzung mit einer wasserfreien Säure mit einem pks-Wert von <0 erhält.

3. Verfahren nach Anspruch 1 und/oder 2,
**dadurch gekennzeichnet, dass**
man Chlorhexidin-Base als Katalysator in einer Menge von 0,0001 Mol% - 0,1 Mol%, bevorzugt 0,001 Mol% - 0,05 Mol% und besonders bevorzugt von 0,01 ± 0,005 Mol% bezogen auf eingesetztes Hexamethylendiamin einsetzt.

4. Verfahren nach einem oder mehreren der vorhergehenden Ansprüche,
**dadurch gekennzeichnet, dass**
man die Umsetzung bei einer Temperatur von 100°C bis 140°C, vorzugsweise 105°C bis 130°C und ganz besonders bevorzugt von 120 ± 10°C betreibt.

5. Verfahren nach einem oder mehreren der vorhergehenden Ansprüche,
**dadurch gekennzeichnet, dass**
man als alkoholisches Lösungsmittel Alkohole ausgewählt aus der Gruppe bestehend aus Methanol, Ethanol, Propanol und Isopropanol oder deren Gemische einsetzt.

6. Verfahren zur Herstellung von Chlorhexidin-Base der Formel (II), 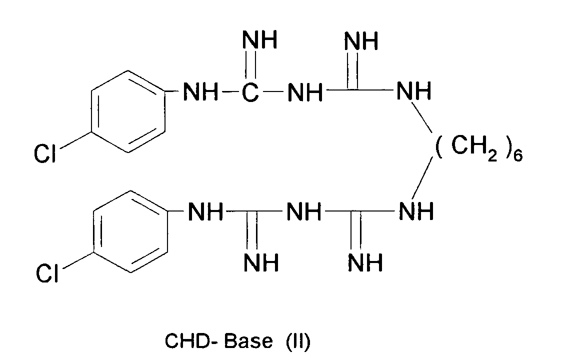 **dadurch gekennzeichnet, dass**
man das HMBCG gemäß Anspruch 1 darstellt und dieses anschließend mit p-Chloranilin umsetzt, wobei man ohne Zwischenisolierung des HMBCGs arbeitet.

## Claims

1. Process for preparing hexamethylenebiscyanoguanidine of the formula (I) 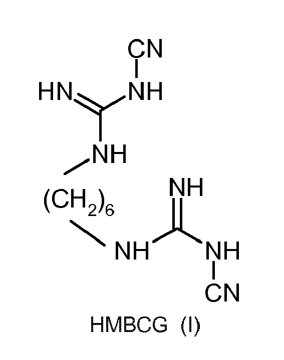 by reacting hexamethylenediamine or its salt with sodium dicyanamide in an alcoholic solvent in the presence of catalytic amounts of chlorhexidine base in one process step.

2. Process according to Claim 1,
**characterized in that**
the salt of hexamethylenediamine is obtained through its reaction with an anhydrous acid with a pKa value of < 0.

3. Process according to Claim 1 and/or 2,
**characterized in that**
chlorhexidine base is used as catalyst in an amount of 0.0001 mol% - 0.1 mol%, preferably 0.001 mol%-0.05 mol% and particularly preferably of 0.01 ± 0.005 mol%, based on hexamethylenediamine used.

4. Process according to one or more of the preceding claims,
**characterized in that**
the reaction is carried out at a temperature of 100°C to 140°C, preferably 105°C to 130°C, and very particularly preferably of 120 ± 10°C.

5. Process according to one or more of the preceding claims,
**characterized in that**
alcohols selected from the group consisting of methanol, ethanol, propanol and isopropanol or mixtures thereof are used as alcoholic solvent.

6. Process for preparing chlorhexidine base of the formula (II) 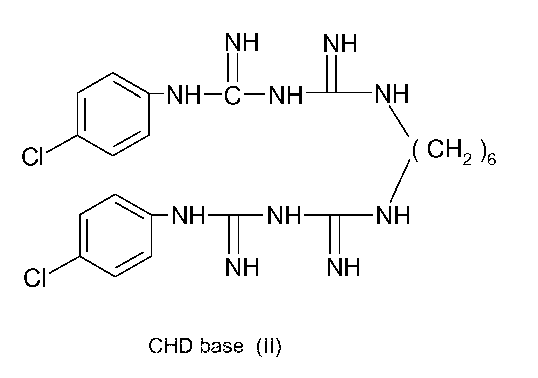 **characterized in that**
the HMBCG is synthesized according to Claim 1 and this is then reacted with p-chloroaniline, the process being carried out without intermediate isolation of the HMBCG.

## Revendications

1. Procédé pour la préparation d'hexaméthylènebiscyanoguanidine de formule (I) 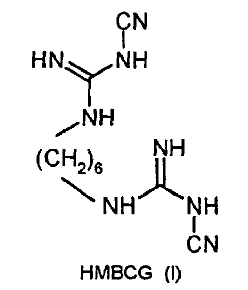 par transformation d'hexaméthylènediamine ou d'un sel avec du dicyanamide sodique dans un solvant alcoolique en présence de quantités catalytiques de chlorhexidine-base en une étape de procédé.

2. Procédé selon la revendication 1, **caractérisé en ce qu'**on obtient le sel de l'hexaméthylènediamine par sa transformation avec un acide anhydre présentant une valeur pka < 0.

3. Procédé selon la revendication 1 et/ou 2, **caractérisé en ce qu'**on utilise la chlorhexidine-base comme catalyseur en une quantité de 0,0001% en mole - 0,1% en mole, de préférence de 0,001% en mole - 0,05% en mole et de manière particulièrement préférée de 0,01 ± 0,005% en mole par rapport à l'hexaméthylènediamine utilisée.

4. Procédé selon l'une ou plusieurs des revendications précédentes, **caractérisé en ce qu'**on réalise la transformation à une température de 100°C à 140°C, de préférence de 105°C à 130°C et de manière tout particulièrement préférée de 120 ± 10°C.

5. Procédé selon l'une ou plusieurs des revendications précédentes, **caractérisé en ce qu'**on utilise, comme solvant alcoolique, des alcools choisis dans le groupe constitué par le méthanol, l'éthanol, le propanol et l'isopropanol ou leurs mélanges.

6. Procédé pour la préparation de chlorhexidine-base de formule (II), 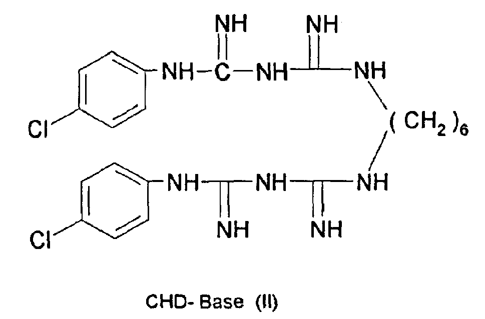 **caractérisé en ce qu'**on prépare le HMBCG selon la revendication 1 et on transforme celui-ci ensuite avec de la p-chloroaniline, en travaillant sans isolement intermédiaire de l'HMBCG.
